# EUROPÄISCHE PATENTSCHRIFT

(11) **EP 4 370 192 B1**
(45) Veröffentlichungstag und Bekanntmachung des Hinweises auf die Patenterteilung: **22.04.2026**
(21) Anmeldenummer: 22751349.6
(22) Anmeldetag: 13.07.2022
(51) Int. Cl.: A61M 39/28

(54) **INFUSIONSBESTECK MIT ROLLENKLEMME**
INFUSION SET HAVING ROLLER CLAMP
ENSEMBLE DE PERFUSION AYANT UNE PINCE À ROULEAUX

(30) Priorität: 15.07.2021 DE 102021118331
(43) Veröffentlichungstag der Anmeldung: 22.05.2024
(73) Patentinhaber: B. Braun Melsungen AG, 34212 Melsungen (DE)
(72) Erfinder: BOLZ, Johannes, 34132 Kassel (DE); KRAMER, Matthias, 34212 Melsungen (DE); FREITAG, Claudia, 34281 Gudensberg (DE); GUHL, Torben, 34119 Kassel (DE)
(74) Vertreter: Winter, Brandl - Partnerschaft mbB
(86) Internationale Anmeldenummer: PCT/EP2022/069646
(87) Internationale Veröffentlichungsnummer: WO 2023/285556

(56) Entgegenhaltungen:
- US-A- 4 725 037
- US-A- 5 259 587
- US-A1- 2003 114 802
- US-A1- 2004 164 258

## Beschreibung

### Technisches Gebiet

Die vorliegende Offenbarung betrifft ein Infusionsbesteck mit einer Rollenklemme zum Einstellen eines Querschnitts eines Schlauchs zur Regulierung einer Tropfrate einer Flüssigkeit, die durch den Schlauch läuft.

### Stand der Technik

Um eine Infusion bei einem Patienten zu legen, werden sogenannte Infusionsbestecke benutzt. Die Infusionsbestecke bestehen aus einer Schnittstelle für die Verbindung mit einem Container (Tropfkammer/ Spike), einem Patientenanschluss zur Ankopplung an eine(n) Patientenschnittstelle (Patientenzugang), einem Schlauch zum Transport/Leitung einer Flüssigkeit, sowie einer Rollenklemme, um die Durchflussgeschwindigkeit durch den Schlauch einzustellen. Ein gängiger Abstand der Rollenklemme zum Container (Tropfkammer) beträgt ca. 25 cm. Dieser Auslieferungszustand bietet einen guten Komfort für die meisten Benutzenden, da ein Flüssigkeitsbehälter, an welchem das Infusionsbesteck angeschlossen ist, in der Regel über Kopf an einem Stativ aufgehängt ist und sich somit die Rollenklemme in einer für eine Bedienperson bequem zugänglichen Höhe anordnet. Die Rollenklemme wird im offenen Zustand ausgeliefert.

Rollenklemmen, die den Durchfluss einer Flüssigkeit durch den elastischen Schlauch begrenzen, sind aus den folgenden Patentschriften bekannt.

US 3 900 184 A offenbart eine Rollenklemme zum Komprimieren eines elastischen Schlauchs. Diese Rollenklemme weist ein Gehäuse und ein Rädchen auf, das in dem Gehäuse dreh- und verschiebbar gelagert ist. Das Rädchen komprimiert/ presst den Schlauch, um den Durchfluss einer Flüssigkeit durch den Schlauch zu reduzieren. Dazu ist eine gegenüber zu dem Rädchen in dem Gehäuse positionierte Auflagefläche für den Schlauch rampenförmig ausgebildet, sodass ein Spiel/Abstand zwischen Schlauch und Rampe immer kleiner wird und der Schlauch folglich immer weiter komprimiert wird, je weiter das Rädchen längs des Schlauchs/längs der rampenförmigen Auflagefläche verschoben wird.

Aus EP 1 452 202 A1 ist ebenfalls eine Rollenklemme bekannt. Diese Rollenklemme weist ein Rollenklemmengehäuse und ein in dem Rollenklemmengehäuse längsverschiebbar und drehbar gelagertes Rädchen auf. Das Rädchen ist an seiner Umfangsseite ausgeschnitten. Das Rollenklemmengehäuse weist eine Aussparung auf, die zu dem ausgeschnittenen Rädchen passt. Gemeinsam bilden das Rollenklemmengehäuse und das Rädchen einen Raum in den der Schlauch gepresst wird. Die Aussparung wird in Längsrichtung des Rollenklemmengehäuses und In Längsrichtung des darin eingelegten Schlauchs kleiner/enger. Damit wird der Raum für den Schlauch kleiner und der Schlauch wird stärker komprimiert, wodurch der Durchfluss durch den Schlauch begrenzt werden kann.

Die Veröffentlichungen US 8 544 815 B2, GB 1 212 781 A, US 4 660 802 A, US 5 352 214 A haben im Wesentlichen die gleiche Funktion wie die vorstehend genannten Offenbarungen, nämlich den Durchfluss einer Flüssigkeit durch einen Flüssigkeits-Leitungsschlauch zu regulieren, haben aber einen zueinander unterschiedlichen Aufbau.

Aus der US 4 725 037 A ist eine Rollenklemme mit einem Rädchen bekannt, das in einer Nut eines Gehäuses beweglich gelagert ist. Beim Verschieben des Rädchens wird ein Schlauch ein- oder abgeklemmt, der in die Rollenklemme eingelegt ist. Die Rollenklemme soll ein komplettes Abklemmen des Schlauches ermöglichen, ohne dass der Schlauch reißt oder beschädigt wird. Die US 4 725 037 A löst dies durch eine genaue Ausgestaltung von Vorsprüngen, die von einer Gehäuseseitenwand in eine Schlauchaufnahme vorstehen.

Problematisch an den bestehenden Lösungen ist, dass der Einstellweg für eine oder im Bereich einer niedrige(n) Tropfrate geringer ist als der Einstellweg für eine oder im Bereich einer größere(n) Tropfrate. In anderen Worten ausgedrückt wurde festgestellt, dass eine Längsverstellung eines Rädchens im Bereich einer niedrigeren Tropfrate eine größere Auswirkung auf die Tropfratenänderung hat als im Bereich einer höheren Tropfrate. Dadurch ist es für einen Nutzer komplizierter, eine geringe Tropfrate genau einzustellen, als das Einstellen einer größeren Tropfrate ist. Des Weiteren sind die Kräfte, die zum Einstellen einer geringen Tropfrate benötig werden, relativ groß, sodass es für den Nutzer unhandlich ist, die Tropfrate einzustellen.

Herkömmliche Rollenklemmen können zum Teil also nur schwer betätigt und schlecht eingestellt werden. Ziel ist es daher grundsätzlich, die Nutzerfreundlichkeit zu verbessern. Dies kann beispielsweise durch eine weitere Spreizung des Einstellbereichs zwischen 0-60 Tropfen/Minute (Bereich niedrigerer Tropfrate) erreicht werden. Ebenso unterliegen Infusionsbestecke dieser Gattung einem hohen Kostendruck, da es sich hierbei im Wesentlichen um Einmal-Artikel handelt. Daher wird gleichzeitig eine Reduzierung der Herstellungskosten angestrebt. Einsparungen können u. a. durch einen geringeren Materialverbrauch, höheren Ausstoß und geringere Wartungskosten erreicht werden. Ebenso werden Materialien bevorzugt, die eine Rezyklierbarkeit ermöglichen. Durch Optimierung des Bauteildesigns kann die Funktionseinheit Rollenklemme günstiger hergestellt werden. Gleichzeitig wird die Funktionsweise optimiert.

Die Rädchen von herkömmlichen Rollenklemmen sind ferner schwer verstellbar und hohen Kräften beim Bewegen/Verstellen ausgesetzt. Deshalb können die Rädchen beschädigt werden oder brechen. Dies kann durch massive Rädchen verhindert werden, die aber dann ungünstige Materialansammlungen aufweisen und viel Material bei der Herstellung benötigen.

### Zusammenfassung der Offenbarung

Der Offenbarung liegt in Anbetracht dieses Fachwissens die Aufgabe zugrunde, die Nachteile bestehender Lösungen zu verbessern und ein Infusionsbesteck mit einer Rollenklemme mit möglichst wenig Krafteinwirkung auf ein Rädchen der Rollenklemme bereitzustellen.

Offenbarungsgemäß wird diese Aufgabe durch ein Infusionsbesteck mit einer Rollenklemme zum Einstellen der Tropfrate an einem Schlauch nach Anspruch 1 gelöst. Vorteilhafte Weiterbildungen der Offenbarung sind Gegenstand der beigefügten Unteransprüche.

Die vorliegende Offenbarung betrifft nach einem ggf. unabhängig beanspruchbaren Aspekt ein Infusionsbesteck mit einem Schlauch und einer Rollenklemme, die an dem Schlauch angebracht ist und mit der eine Tropfrate/ ein Durchfluss durch den Schlauch reduziert werden kann. Hierfür weist die Rollenklemme ein im Querschnitt annähernd U-/ oder C-förmiges Gehäuse auf. Der Schlauch liegt im Gehäuse auf einem die Seitenflanken des U--/ oder C-förmigen Gehäuse vorzugsweise verbindenden Steg auf. Ein Rädchen ist an Laufflächen in oder an den Seitenwänden des Gehäuses drehbar und in Längsrichtung des Gehäuses verschiebbar abgestützt, um durch eine Bewegung/Drehung des Rädchens längs der Laufflächen einen Querschnitt des Schlauchs zu verändern. Die Laufflächen sind dabei dem Steg zugewandt und erstrecken sich längs/in ihrer Länge in Gehäuselängsrichtung und quer/in ihrer Breite in Gehäusequerrichtung. Die Laufflächen sind zu einer Rotationsachse des Rädchens derart angestellt, dass die Laufflächen jeweils eine im Wesentlichen punktförmige Abstütz- oder Kontaktstelle mit einer Radachse des Rädchens bilden, die in einem Innenabschnitt der Radachse ausgebildet ist. Der Innenabschnitt ist in Breitenrichtung gesehen einer Radmitte des Rädchens zugewandt. Vorzugsweise ist der Innenabschnitt der Laufflächen zu dem Rädchen, also zur Innenseite des Gehäuses hin ausgerichtet.

Das Rädchen liegt in den Seitenwänden des Gehäuses an den Laufflächen an. Die Laufflächen erstrecken sich in Längsrichtung über einen gesamten Verstellweg des Rädchens. In Breitenrichtung erstrecken sich die Laufflächen quer zur Gehäuselängsrichtung über die Breite der Seitenwände oder über einen Teil der Breite der Seitenwände. Im Innenabschnitt der Laufflächen ist die Abstütz-, Auflage- oder Kontaktstelle zwischen dem Gehäuse und dem Rädchen bzw. der Radachse des Rädchens ausgebildet.

Die im Wesentlichen punktförmige Abstütz- oder Kontaktstelle zwischen einer runden/zylindrischen Achse des Rädchens und der (linienförmigen) Lauffläche ist lediglich theoretisch punktförmig. In Wirklichkeit kann die Abstütz- oder Kontaktstelle durch (minimale) Deformation der beteiligten Elemente eine Fläche sein. Im Sinne dieser Offenbarung soll unter einer punktförmigen Abstütz- oder Kontaktstelle auch eine durch (minimale) Deformation entstandene Fläche verstanden werden.

Das Klemmgehäuse weist eine Gehäuselauffläche auf, in oder an der das Rädchen entlangläuft oder abgestützt ist. Das Rädchen weist ebenfalls eine Radlauffläche auf, die in Kontakt mit der Gehäuselauffläche steht. Die Radlauffläche steht nicht über die gesamte Breite in Kontakt mit der Gehäuselauffläche, sondern nur in einem begrenzten Kontaktbereich. Somit ist zumindest ein Teil der Radlauffläche freigestellt. Die Freistellung des Kontaktbereichs zwischen Rädchen und Gehäuse(-Auflagefläche) ist derart ausgebildet, dass der Kontaktbereich zwischen der Radlauffläche und der Gehäuselauffläche zur Radmitte verschoben ist. In anderen Worten ist die Lauffläche des Rades derart freigestellt, dass eine Kontaktstelle zwischen dem Gehäuse und dem Rad in Richtung eines Radmittelpunktes verschoben ist.

In anderen Worten sind die Laufflächen des Klemmgehäuses derart, insbesondere sich nach innen hin verjüngend, angestellt, dass die (punktförmige) Abstütz- oder Kontaktstelle in dem Innenabschnitt der jeweiligen Laufflächen ausgebildet ist. D.h. der Abstand zwischen dem Steg und der Lauffläche kann in einem äußeren Abschnitt maximal sein und sich zur Gehäusemitte hin verringern. Dadurch kann die Abstütz- oder Kontaktstelle am innersten Abschnitt der jeweiligen Laufflächen positioniert sein. Somit kann eine Belastung auf das Rädchen bzw. eine mögliche elastische Deformation der Radachse vermindert werden.

Durch den begrenzten Kontaktbereich wird eine mögliche elastische Deformation in der Radachse reduziert. Die Belastung auf das Rädchen ist durch den zur Radmitte verschobenen Kontaktbereich geringer. Es ist beispielsweise sinnvoll, die Belastung auf die Achse des Rädchens zu reduzieren, wenn die Wandstärke der Radachse durch eine Aushöhlung reduziert ist.

Vorzugsweise ist die Abstütz- oder Kontaktstelle an der maximal innersten Stelle der jeweiligen Lauffläche ausgebildet. Dadurch kann der Abstand zwischen einem Schwerpunkt des Rädchens und der Abstütz- oder Kontaktstelle minimal werden. Somit kann auch die Belastung an den Außenseiten des Rädchens bzw. der Radachse minimal werden.

Vorzugsweise ist die Radachse des Rädchens konisch ausgebildet, sodass die (punktförmige) Abstütz- oder Kontaktstelle in dem Innenabschnitt der jeweiligen Laufflächen ausgebildet ist. Eine konische Radachse könnte die Abstütz- oder Kontaktstelle in Richtung der Radmitte verschieben, wenn die Laufflächen an oder in den Seitenwänden des Gehäuses plan ausgebildet sind. Somit können die Vorteile der nach innen verlagerten Abstütz- oder Kontaktstelle auch bei einer planen Gehäuselauffläche ausgenutzt werden. Es ist natürlich ebenfalls denkbar, dass sowohl die Radachse konisch ausgebildet ist, als auch die Laufflächen des Gehäuses angestellt ist.

Bei einer vorteilhaften Ausgestaltung der Offenbarung, die in Kombination mit anderen Merkmalen oder auch alleinstehend ausgestaltet sein kann, ist vorgesehen, dass die Radachse des Rädchens hohl ausgebildet ist. Insbesondere kann das Rädchen beidseitig ausgebildete Achszapfen aufweisen, die in die Lauffläche des Klemmgehäuses eingreifen. In einer Seite der Achszapfen kann eine Bohrung angebracht sein, die die Achszapfen (teilweise) aushöhlt.

Anders ausgedrückt ist die Radachse des Rädchens nicht massiv gefertigt, sondern weitestgehend hohl. Die Aushöhlung verläuft entlang der Rotationsachse des Rädchens von einer Seite der Radachse zur anderen Seite der Radachse. Dadurch ist die Wandstärke des Rädchens reduziert. Durch die Aushöhlung ist das Rädchen spritzgussgerecht gestaltet. Die Radachse stellt damit keine Materialansammlung dar, in der sich Lunker und/ oder Luftblasen bilden können. Das Rädchen verbraucht weniger Material als ein Rädchen ohne die Aushöhlung und eine Zykluszeit beim Spritzgießen wird reduziert.

Die Verminderung der Belastung auf das Rädchen bzw. auf die Radachse durch die Positionierung der Abstütz- oder Kontaktstelle kann eine Voraussetzung dafür sein, die Radachse hohl zu gestalten. Dadurch könnte auch die hohle Radachse den beim Verschieben des Rädchens entstehenden Kräften standhalten.

Das Einstellverhalten der Rollenklemme soll ferner derart angepasst werden, dass der (Teil-)Weg für die Einstellung niedriger Tropfraten vergrößert wird. Gleichzeitig soll der (Gesamt-)Weg für die Einstellung von dem geschlossenen Zustand zum offenen Zustand (Free Flow) gleich lang oder kürzer als bisher sein. Das Verhalten bezüglich der Tropfratenkonstanz soll mindestens gleichbleiben. Die Verstellkräfte sollen so gering wie möglich sein.

Der Schlauch kann im Gehäuse auf einem die Seitenflanken des U-/oder C-förmigen Gehäuse vorzugsweise verbindenden Steg mit einer längs des Gehäuses sich erstreckenden Schlauchauflage-Kulisse/Aussparung/Nut aufliegen. In/an den Seitenflanken/Seitenwänden des Gehäuses ist ein Rad drehbar und längsverschieblich gelagert, welches sich gegen den Schlauch abstützen kann und diesen somit gegen den Steg in/an die Schlauchauflage-Kulisse ein-/andrücken kann. Durch eine Bewegung/Drehung des Rades kann sich dieses längs des Schlauchs auf diesem abwälzen, wodurch der Schlauch von dem Rad fortlaufend in die Kulisse gepresst werden kann. Dabei kann die Breite der Kulisse in Längsrichtung des Gehäuses stetig abnehmen. Wenn das Rad also in die Längsrichtung des Gehäuses verschoben/vorwärtsbewegt wird, kann das Rad den Schlauch in die immer schmaler werdende Kulisse fortlaufend hineinpressen. Dadurch kann eine Pressung auf den Schlauch innerhalb der Kulisse stetig erhöht und der Durchfluss durch den Schlauch stetig vermindert werden.

Die Kulisse kann einen (Längs-)Abschnitt im Bereich einer geringeren Schlauchpressung (mit fortlaufend zunehmender Schlauchpressung) und einen daran sich anschließenden (Längs-)Abschnitt im Bereich einer größeren Schlauchpressung (mit fortlaufend zunehmender Schlauchpressung) aufweisen. Der Abschnitt im Bereich der größeren Schlauchpressung kann länger sein als der Abschnitt im Bereich der geringeren Schlauchpressung.

In anderen Worten ausgedrückt kann der Gesamt-Verstellweg des Rädchens längs des in das Gehäuse eingelegten Schlauchabschnitts/ längs einer dem Rädchen gegenüberliegenden Schlauchauflagefläche des Gehäuses (in der eine längsverlaufende Schlauchquetschnut / Schlauchquetsch-Kulisse ausgeformt ist) in zwei Teilverstellwege unterteilt sein, die sich längs des Gesamt-Verstellwegs unmittelbar aneinanderschließen. Der erste Teilverstellweg kann dabei einen Bereich betreffen, in welchem der Schlauch auf einem Niveau höherer Tropfrate zunehmend (vorzugsweise kontinuierlich zunehmend) komprimiert, wohingegen der zweite Teilverstellweg einen Bereich betreffen kann, in welchem der Schlauch auf einem Niveau kleinerer Tropfrate zunehmend (vorzugsweise kontinuierlich zunehmend) komprimiert wird. Der zweite Teilverstellweg ist vorzugsweise länger als der erste Teilverstellweg dimensioniert. Vorzugsweise ist die Steigung (Tropfratenänderung pro Verstellweg-Einheit) im ersten Teilverstellweg größer als im zweiten Teilverstellweg.

Dabei ist der Abschnitt (im Bereich) der geringeren Schlauchpressung vorzugsweise ein Abschnitt mit (tendenziell) größerer Tropfrate und der Abschnitt (im Bereich) der größeren Schlauchpressung ist ein Abschnitt mit (tendenziell) geringerer Tropfrate. Die Verstellkraft, die nötig ist, das Rad zu bewegen und den Schlauch zu pressen bzw. die gewünschte Tropfrate durch den Schlauch einzustellen, kann von der Pressung des Schlauches auf den Boden abhängen.

Auf der einen Seite ist die Verstellkraft derart eingestellt, dass der Benutzer das Rädchen leicht verschieben kann, den Schlauch also ergonomisch pressen kann Auf der anderen Seite kann die Verstellkraft auch so eingestellt sein, dass der Benutzer ein hochwertiges Gefühl beim Verschieben hat. D.h. die nötige Verstellkraft darf nicht zu gering sein und das Rädchen darf nicht zu einfach zu verschieben sein.

Der Verstellweg, den das Rad zurücklegen muss, um eine gewünschte Tropfrate einzustellen, kann von der (Gesamt-)Länge der Kulisse abhängen. Da der Abschnitt der größeren Schlauchpressung vorzugsweise länger als der Abschnitt der geringeren Schlauchpressung ist, ist der Verstellweg/ Einstellweg der geringeren Tropfrate vorzugsweise länger als bei herkömmlichen Rollenklemmen. Da der Abschnitt der geringeren Schlauchpressung jedoch kürzer als bei herkömmlichen Rollenklemmen ist, kann es möglich sein, die Gesamtlänge der Kulisse im Vergleich zu herkömmlichen Rollenklemmen nicht zu verändert, sodass der Gesamtweg von einem offenen zu einem geschlossenen Zustand der Rollenklemme der gleiche sein kann.

In anderen Worten ausgedrückt liegt der Schlauch vorzugsweise auf dem Steg des Gehäuses auf. Damit bildet der Steg eine Schlauchauflagefläche. Der Schlauch wird von dem Rädchen, dass in einer Nut in der Seitenwand in Längsrichtung des Gehäuses beweglich gelagert ist, vorzugsweise zusammengedrückt/ komprimiert/ gepresst. Dabei kann der Schlauch in eine Kulisse/Längsnut gepresst werden. Die Breite/Tiefe der Kulisse kann den verbleibenden Querschnitt des Schlauchs bestimmen. Der verbleibende Querschnitt des Schlauchs wiederum beeinflusst vorzugsweise die Tropfrate/ den Durchfluss einer Flüssigkeit, die durch den Schlauch fließt. D.h. die Geometrie der Kulisse beeinflusst vorzugsweise die Durchflussrate/ Tropfrate der Flüssigkeit in dem Schlauch. Dabei kann die Kulisse derart ausgebildet sein, dass die Kulissenbreite/-Tiefe in der Längsrichtung des Klemmgehäuses (kontinuierlich) abnimmt. An einer Vorderseite des Klemmgehäuses kann die Kulisse ihre maximale Breite aufweisen und wird dann vorzugsweise zur anderen Seite hin immer schmaler. Wenn das Rädchen in Längsrichtung des Klemmgehäuses verschoben wird, kann es den Schlauch also immer weiter in die immer schmaler werdende Kulisse einklemmen und kann so den Querschnitt des Schlauches vermindern. Die minimale Kulissenbreite ist dabei vorzugsweise derart gewählt, dass keine Flüssigkeit durch den Schlauch hindurchfließt, die Tropfrate bei der minimalen Kulissenbreite also vorzugsweise null ist.

Durch die Verminderung der Kulissenbreite kann die Kulisse im Wesentlichen eine Trapezform haben. Dabei ist die Verringerung der Kulissenbreite aber nicht konstant, sondern die Kulisse weist im Wesentlichen zwei Abschnitte mit einer jeweils konstanten Verringerung und einem Knickpunkt zwischen den zwei Abschnitten auf. Dabei ist der erste Abschnitt der Abschnitt für das Einstellen der großen Tropfrate und der zweite Abschnitt der Abschnitt für das Einstellen der kleinen Tropfrate. Der Abschnitt der kleineren Tropfrate ist dabei größer als der Abschnitt der größeren Tropfrate. In anderen Worten ausgedrückt, ist die Flanke der Kulisse im Abschnitt der kleineren Tropfrate weniger steil als im Abschnitt der größeren Tropfrate. Dadurch ist der Verstellweg im Abschnitt der kleineren Tropfrate größer als der Verstellweg im Abschnitt der großen Tropfrate und eine kleine Tropfrate kann genauer eingestellt werden.

Durch die Ausgestaltung der Kulisse kann der Einstellweg für niedrige Tropfraten vergrößert werden, dabei kann aber der gesamte Einstellweg von einem geschlossenen zu einem komplett offenen Zustand nicht vergrößert werden. Des Weiteren sind durch die geänderte Kulissengeometrie die zum Verstellen der Tropfrate benötigten Kräfte vorzugsweise klein. Dadurch ist ein bequemeres und ergonomischeres Einstellen der Rollenklemme möglich.

Dieser Aspekt kann die folgenden Vorteile bieten:
- Verringerung der Stückkosten durch geringeren Materialverbrauch
- Verbessertes Einstellverhalten bei geringerer Bedienkraft
- Einfacheres Einstellen bei niedrigen Tropfraten.
- Längere Standzeit des Werkzeugs zur Fertigung der Rollenklemme

Der Gesamtverstellweg von dem geschlossenen zu dem offenen Zustand kann von der Länge der Kulisse abhängig sein. Die Kulissenlänge wird durch die Geometrieänderung im Vergleich zu bekannten Rollenklemmen vorzugsweise nicht verändert. Dadurch ändert sich auch der Gesamtverstellweg der Rollenklemme vorzugsweise nicht.

Bei einer vorteilhaften Ausgestaltung der Offenbarung, die in Kombination mit anderen Merkmalen oder auch alleinstehend ausgestaltet sein kann, ist vorgesehen, dass die minimale Kulissenbreite gleich oder kleiner als ein Drittel der größten Kulissenbreite ist. Das Verhältnis von der minimalen zur maximalen Kulissenbreite wirkt sich auf die Steilheit der Flanke der Kulisse und damit auf den Verstellweg des Rädchens aus.

Bei einer vorteilhaften Ausgestaltung der Offenbarung, die in Kombination mit anderen Merkmalen oder auch alleinstehend ausgestaltet sein kann, ist vorgesehen, dass der Steg eine vorzugsweise durch eine im Steg ausgebildete Stufe definierte, sowie in Querrichtung des Gehäuses verlaufende Kante aufweist, wodurch der Schlauch beim Arretieren der Rollenklemme auf dem Schlauch mittels des Rädchens sowohl an der Kante als auch an einem dem Steg gegenüberliegenden Randabschnitt einer Durchgangsöffnung anliegt und somit eingeklemmt ist.

In anderen Worten wird der Schlauch bei der Arretierung der Rollenklemme auf dem Schlauch durch Umlenkung geklemmt. In nochmals anderen Worten ausgedrückt wird die Rollenklemme an dem Schlauch befestigt, indem der Schlauch in/an dem Klemmengehäuse festgeklemmt wird. Der Schlauch wird vorzugsweise festgeklemmt, indem der Schlauch an einer Kante in dem Steg und an einer Durchgangsöffnung in dem Klemmgehäuse umgelenkt wird. Der Schlauchquerschnitt kann dabei nicht oder nur unwesentlich deformiert werden. Dadurch kann der Effekt der Schlauchdeformation durch eine Rändelung des Rädchens stark reduziert und gleichzeitig die benötigte Verschiebekraft für die Positionierung der Rollenklemme auf dem Schlauch reduziert werden.

Vorzugsweise ist die Kante mit Blickrichtung hin zur Durchgangsöffnung ausgebildet.

Bei einer vorteilhaften Ausgestaltung der Offenbarung, die in Kombination mit anderen Merkmalen oder auch alleinstehend ausgestaltet sein kann, ist vorgesehen, dass die Durchgangsöffnung am unteren/stromabwärtigen Ende der Rollenklemme derart ausgebildet ist, dass der Durchmesser der Durchgangsöffnung im Wesentlichen dem Durchmesser des Schlauches entspricht. Anders ausgedrückt ist die Durchgangsöffnung am unteren Ende der Rollenklemme derart groß, dass der Schlauch nicht in der Durchgangsöffnung steckenbleibt, aber gerade derart klein, dass der Schlauch kein Spiel in der Durchgangsöffnung hat. Dadurch ist der Schlauch in eine Richtung quer zur Längsrichtung des Schlauches gelagert. D.h. der Schlauch kann sich nicht senkrecht zu seiner Längsrichtung bewegen. Zusammen mit der Kante im Steg, bewirkt die Lagerung des Schlauches in der Durchgangsöffnung, dass der Schlauch in der Rollenklemme arretiert/ fixiert/ befestigt ist, ohne, dass der Schlauchquerschnitt durch das Rädchen deformiert werden muss.

Vorzugsweise ist das Rädchen über einen vordefinierten Verschiebeweg in Längsrichtung verschiebbar und die Kante in einem Mittenabschnitt des Verschiebewegs positioniert. Der Verschiebeweg des Rädchens kann in verschiedene Abschnitt aufgeteilt sein. So kann der Verschiebeweg einen Anfangsbereich, in dem die Rollenklemme bezüglich des Schlauchs beweglich ist, den Mittenabschnitt, in dem die Rollenklemme auf dem Schlauch verklemmt ist aber Flüssigkeit durch den Schlauch fließen kann, und einen Endabschnitt aufweisen, in dem keine Flüssigkeit durch den Schlauch fließen kann.

Bei einer vorteilhaften Ausgestaltung der Offenbarung, die in Kombination mit anderen Merkmalen oder auch alleinstehend ausgestaltet sein kann, ist vorgesehen, dass ein Schlauchhalter asymmetrisch ist. Der Schlauchhalter ist eine Ausbuchtung an einer Seite des Gehäuses, die dazu vorbereitet und ausgebildet ist, den Schlauch aufzunehmen. In den Schlauchhalter wird ein Ende des Schlauches geklemmt, meist das Ende, das an den Patienten angelegt wird. Beispielsweise wird der Schlauch in den Schlauchhalter geklemmt, wenn der Schlauch vor dem Anlegen an einen Patienten entlüftet wird. Der Schlauchhalter hat eine tief eingeschnittene Seite und eine flache Seite. In der tief eingeschnittenen Seite kann der Schlauch eingedrückt werden und hat dort einen festen Sitz.

Durch die Schlauchhaltergeometrie ist die Positionierungs- und Bauraumoptimierung angepasst. Das Resultat ist die asymmetrische Geometrie. Die Geometrie wurde dahingehend optimiert, einen festen Sitz bei einfachem Eindrücken und Herausziehen des Schlauches zu ermöglichen. Ein Herausfallen des eingeklemmten Endes des befüllten IV-Sets beim Schütteln des Systems ist nicht möglich. Das einhändige Einhängen und Entfernen des Schlauches ist dagegen problemlos möglich.

Bei einer vorteilhaften Ausgestaltung der Offenbarung, die in Kombination mit anderen Merkmalen oder auch alleinstehend ausgestaltet sein kann, ist vorgesehen, dass das Klemmgehäuse innenseitig eine elastische Rippe aufweist, die einen Dorn eines Schlauches einklemmt. Die Rippe hat die Funktion eines Dornschutzes. Der Dornschutz ist vorzugsweise durch eine 3-Punkt Klemmung realisiert. Dadurch kann die Schlitzung im Klemmgehäuse, die in herkömmlichen Klemmrollen gängig ist, entfallen. Eine Dichtfläche für die Schlitzung kann ebenfalls entfallen.

Da keine Schlitzung des Klemmgehäuses notwendig ist, sind die Seitenwände/ die Unterseite des Klemmgehäuses durchgehend. Anders ausgedrückt weist das Klemmgehäuse im Gegensatz zum Stand der Technik keinen Schlitz in der Seitenwand/Flanke auf. Der Schlitz in der Seitenwand stellt in bekannten Lösungen den Dornschutz dar. Der Dornschutz wird verwendet, um den Schlauch beim Ankoppeln bzw. Entkoppeln von dem Flüssigkeitsbehälter oder Patienten beispielsweise zur Entsorgung des Schlauches zu halten und zu verbergen. Da kein Schlitz in das Klemmgehäuse eingebracht werden muss, ist ein Werkzeug, mit dem das Klemmgehäuse gefertigt wird, weniger beansprucht und es entsteht weniger Verschleiß an dem Werkzeug. Die Klemmung des Schlauchs wird durch eine 3-Punkt-Klemmung realisiert. Der Querschnitt des Klemmgehäuses ist wie ein Halbkreis und ein Punkt gestaltet. Das elastische Element ist dabei die Rippe. In der Folge ist ein Sitz fester als beim Stand der Technik und die Lösung hat den Vorteil, dass die Dichtfläche für die Schlitzung entfallen konnte. In anderen Worten ausgedrückt ist der Dornschutz zum kompletten Einklemmen des Dorns im Inneren des Klemmgehäuses angebracht. Der Schlauch wird innerhalb des Klemmgehäuses festgeklemmt.

Bei einer vorteilhaften Ausgestaltung der Offenbarung, die in Kombination mit anderen Merkmalen oder auch alleinstehend ausgestaltet sein kann, ist vorgesehen, dass in der Seitenwand eine Radabhebekulisse eingebracht ist, die es dem Rädchen ermöglicht, sich von der Schlauchauflagefläche abzuheben.

Anders ausgedrückt ermöglicht die Radabhebekulisse dem Rädchen nach oben von der Schlauchauflagefläche auszuweichen. Beim Öffnen der Rollenklemme bewegt sich das Rädchen zur oberen Seite der Rollenklemme. Ohne die Radabhebekulisse würde das Rädchen der Kontur der Schlauchauflagefläche folgen. Durch die Pressung des Rädchens auf den Schlauch könnte das Rädchen unter einem Gehäusesteg aus dem Klemmgehäuse gedrückt werden. Durch die Radabhebekulisse hebt das Rädchen beim Öffnen der Klemmrolle aus der Nut ab und verlässt somit die Kontur der Schlauchauflagefläche. In Konsequenz wird der Kontakt des Rädchens zum Schlauch und damit die Möglichkeit des Rädchens, sich auf dem Schlauch abzustützen reduziert. Dadurch kann das Rad nicht mehr unter dem begrenzenden Gehäusesteg aus dem Gehäuse gedrückt werden.

Bei einer vorteilhaften Ausgestaltung der Offenbarung, die in Kombination mit anderen Merkmalen oder auch alleinstehend ausgestaltet sein kann, ist vorgesehen, dass das Klemmgehäuse in der Mitte eine Stufe auf der Rückseite des Klemmgehäuses aufweist. Die Stufe erzeugt ein ergonomisches Halten der Klemme und ein einfaches Schließen. Bezogen auf die Größe wird derart ein optimales Zusammenspiel erzeugt.

Bei einer vorteilhaften Ausgestaltung der Offenbarung, die in Kombination mit anderen Merkmalen oder auch alleinstehend ausgestaltet sein kann, ist vorgesehen, dass die Länge und die Breite des Klemmgehäuses im Vergleich zum Stand der Technik verringert sind. Dadurch wird Material eingespart und Kosten gesenkt.

Vorzugsweise weist das Klemmgehäuse in der Seitenwand eine Nut auf, die sich in Längsrichtung des Klemmgehäuses erstreckt. Die Nut soll Biegekräften in einem Radeinbringungsbereich des Klemmgehäuses reduzieren. Bei einer Montage des Rädchens in das Klemmgehäuse kann der Radeinbringungsbereich aufgebogen werden, damit eine Achse des Rädchens innerhalb einer Einführrampe und einer Stufe zum Liegen kommen kann. Das Rädchen kann also unter (temporärer) Deformation des Klemmgehäuses entlang der Schlauchachse in das Klemmgehäuse eingedrückt werden. Die längliche Nut kann die Kraft reduzieren, die nötig sein kann, um den Radeinbringungsbereich zu verbiegen. Damit kann die längliche Nut eine (manuelle) Montage erleichtern. Somit können Montagedauer und -kosten verringert werden.

Bei einer vorteilhaften Ausgestaltung der Offenbarung, die in Kombination mit anderen Merkmalen oder auch alleinstehend ausgestaltet sein kann, ist vorgesehen, dass das Volumen des Klemmgehäuses weniger als 301 mm³ beträgt. Es ist ferner vorgesehen, dass das Volumen des Klemmrollenrädchens (10) weniger als 321 mm³ beträgt.

Bei einer vorteilhaften Ausgestaltung der Offenbarung, die in Kombination mit anderen Merkmalen oder auch alleinstehend ausgestaltet sein kann, ist vorgesehen, dass die Länge des Klemmgehäuses weniger als 54 mm beträgt. Es ist ferner vorgesehen, dass die Breite des Klemmgehäuses weniger als 15 mm beträgt. Die Höhe des Klemmgehäuses beträgt weniger als 24 mm.

Zusammenfassend wurden durch den offenbarungsgemäßen Gegenstand die folgenden vorteilhaften Effekte erzielt:
- Reduzierung des Materialaufwandes durch Geometrieanpassung (Verkürzung des Gehäuses).
- Reduzierung des Materialaufwandes des Rollenklemmenrädchens durch spritzgussgerechte Auslegung und Anpassung des Materials (ABS)
- Längerer Einstellweg für den Bereich der niedrigen Tropfraten (0-60 Tropfen/min)
- Anpassung des Klemmmechanismus für den Schlauch in der Parkposition (Auslieferungszustand): Vermeidung von sichtbaren Deformationen auf dem Schlauch. Der Schlauch wird nicht geklemmt, sondern umgelenkt.
- Vereinfachung des Werkzeugs (Verschleißsicherer) durch Schließen der Aussparung beim Dornschutz.
- Einbringung einer Radabhebekulisse um das Herausspringen des Rollenklemmenrädchens zu erschweren.

### Kurzbeschreibung der Figuren

Fig. 1 zeigt eine isometrische Ansicht einer offenbarungsgemäßen Rollenklemme;
Fig. 2 zeigt eine Draufsicht auf ein Klemmgehäuse der Rollenklemme;
Fig. 3a zeigt eine Schnittansicht des Klemmgehäuses durch B - B in Fig. 2;
Fig. 3b zeigt einen Ausschnitt D in Fig. 3a;
Fig. 3c zeigt eine Schnittansicht des Klemmgehäuses durch F - F in Fig. 2;
Fig. 3d zeigt einen Ausschnitt G in Fig. 3c;
Fig. 3e zeigt eine Schnittansicht des Klemmgehäuses durch C - C in Fig. 2;
Fig. 3f zeigt einen Ausschnitt E in Fig. 3e;
Fig. 4 zeigt eine Geometrie einer Kulisse in der Draufsicht;
Fig. 5a zeigt eine Seitenansicht des Klemmgehäuses;
Fig. 5b zeigt einen Querschnitt durch das Klemmgehäuse;
Fig. 6a zeigt eine Draufsicht auf ein Rädchen;
Fig. 6b zeigt eine Schnittansicht des Rädchens durch den Schnitt A - A;
Fig. 7 zeigt einen Querschnitt durch das Klemmgehäuse und das Rädchen;
Fig. 8 zeigt eine Arretierung der Rollenklemme auf einem Schlauch durch Umlenkung des Schlauches;
Fig. 9 zeigt einen Längsschnitt durch die Rollenklemme mit einer Radabhebekulisse;
Fig. 10 zeigt einen Querschnitt durch einen Schlauchhalter;
Fig. 11 zeigt eine Schlauchhaltergeometrie;
Fig. 12 zeigt die Länge des Schlauchhalters;
Fig. 13 zeigt einen Querschnitt durch einen Dornschutz;
Fig. 14 zeigt eine Ansicht einer Unterseite des Klemmgehäuses;
Fig. 15 zeigt eine isometrische Ansicht des Klemmgehäuses;
Fig. 16 zeigt eine schematische Darstellung einer Hand, die die Rollenklemme umfasst; und
Fig. 17 zeigt eine Seitenansicht der Rollenklemme mit Bemaßungen.

### Detaillierte Beschreibung der Figuren

Im Folgenden wird der offenbarungsgemäße Gegenstand anhand der Figuren beschrieben.

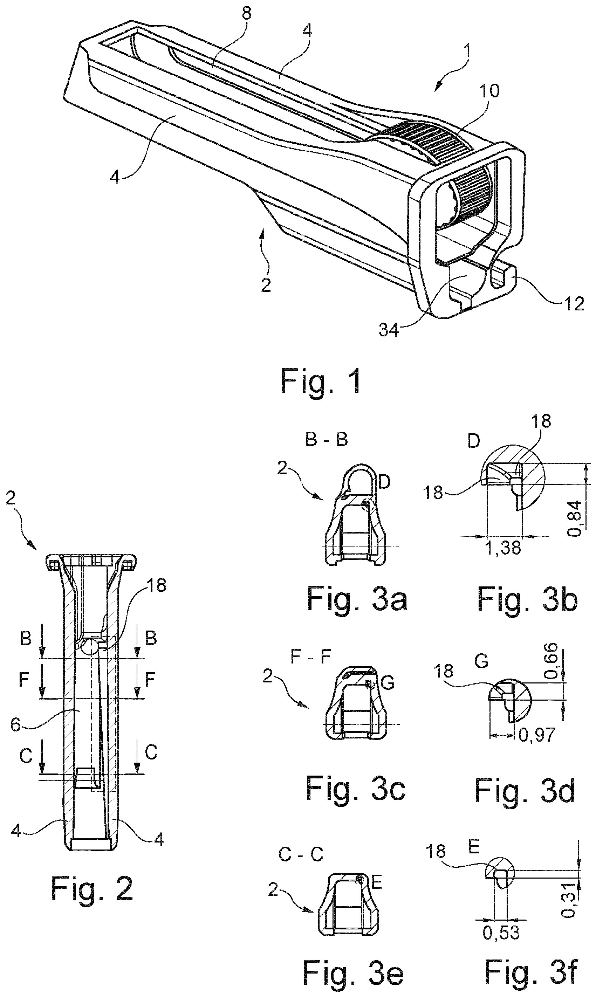

Fig. 1 zeigt eine Rollenklemme 1 zum Anbringen an einen Schlauch. Die Rollenklemme 1 weist ein längliches Klemmgehäuse 2 mit einem im Wesentlichen U oder C-förmigen Querschnitt mit zwei gegenüberliegenden Seitenwänden/Flanken 4 und einem Steg 6 auf, der die zwei Seitenwände 4 verbindet. In oder an jeder Seitenwand verläuft eine Lauffläche 8, bzw. jede Seitenwand ist derart geformt, dass eine solche Lauffläche 8 entsteht, die dafür vorgesehen und ausgebildet ist, ein Rädchen 10 drehbar und längsverschieblich aufzunehmen. Die Lauffläche 8 verläuft (im Wesentlichen) parallel zu dem Steg 6 bzw. zu einer von diesem ausgebildeten Schlauchauflagefläche in Längsrichtung des Klemmgehäuses 2. Die Rollenklemme ist dafür vorbereitet und ausgebildet, an dem Schlauch 14 befestigt zu werden. Der Schlauch 14 verläuft dazu auf dem Steg 6 (auf der Schlauchauflagerfläche). Das Klemmgehäuse 2 weist ferner einen Schlauchhalter 12 und einen Dornschutz 34 auf. Der Schlauchhalter 12 und der Dornschutz 34 werden nachfolgend detaillierter beschrieben.

Die Rollenklemme 1 wird derart an dem Schlauch 14 befestigt, dass ein Längs-Ende der Rollenklemme 1, das näher an dem Rädchen 10 positioniert ist, nach oben/stromauf zu einer Tropfkammer zeigt und das andere Längs-Ende, das von dem Rädchen 10 entfernt ist, nach unten/stromab in Richtung hin zu einem Patienten zeigt. Deshalb wird im Folgenden das Ende mit dem Rädchen 10 als ein oberes Ende und das Ende, das weiter von dem Rädchen 10 entfernt ist, als ein unteres Ende bezeichnet.

Fig. 2 zeigt eine Draufsicht des Klemmgehäuses 2 ohne das Rädchen 10. Der Steg 6 ist dafür vorbereitet, den Schlauch 14 zu führen. Der Steg 6 bildet dafür die Schlauchauflagefläche 20 aus, auf der der Schlauch 14 aufliegt. Der Steg 6 weist eine Kulisse/Längsnut 18 auf. Die Kulisse 18 verläuft in Längsrichtung des Klemmgehäuses 2 und ist zu einer Seitenwand 4 versetzt. Die Kulisse 18 weist zwei Längs-Enden auf, ein vorderes/oberes Ende ist näher an dem Rädchen 10 und somit näher an dem oberen Ende der Rollenklemme 1. Ein hinteres/unteres Ende ist weiter von dem Rädchen 10 entfernt. Von dem oberen bis zu dem unteren Ende hin wird die Kulisse 18 schmaler/flacher. D.h. das vordere Ende der Kulisse 18 ist das Ende mit einer größten Kulissenbreite/-tiefe während das hintere Ende der Kulisse 18 das Ende mit einer minimalen Kulissenbreite/-tiefe ist.

Wenn die Rollenklemme 1 an dem Schlauch 14 angebracht ist, also der Schlauch 14 in das Klemmgehäuse 2 eingeführt ist, wird der Schlauch 14 von dem Rädchen 10 komprimiert. Der Schlauch 14 wird dabei von dem Rädchen 10 auf die Schlauchauflagefläche 20 gepresst. Dafür wird das Rädchen 10 in Längsrichtung des Klemmgehäuses 2 bewegt. Dabei wird der Schlauch 14 in die Kulisse 18 gepresst. Die Kulisse 18 ist der einzige freie Platz, in dem der Querschnitt des Schlauchs 14 ausweichen kann, sobald eine Druckkraft seitens des Rädchens auf ihn ausgeübt wird. Wenn die Kulisse 18 in Längsrichtung des Klemmgehäuses 2 enger/flacher wird, wird auch der Platz für den Schlauchquerschnitt immer kleiner. Der Schlauch 14 wird also durch die Längsbewegung des Rädchens 10 zum unteren Ende hin immer weiter innerhalb der Kulisse 18 komprimiert. Dadurch beeinflusst die Form der Kulisse 18 den Durchfluss durch den Schlauch 14. Die Kulisse 18 befindet sich seitlich im Klemmbereich. Dadurch wird der Einfluss des Schlauches 14 durch das spannungsinduzierte Fließen minimiert und evtl. Toleranzen des Schlauches 14 besser kompensiert.

Die Figuren 3a - 3f zeigen Querschnitte durch das Klemmgehäuse 2 an verschiedenen Stellen in der Längsrichtung des Klemmgehäuses 2. Dabei verlaufen die Querschnitte von einem hohen Durchfluss durch den Schlauch 14 zu einem niedrigen Durchfluss durch den Schlauch 14. Fig. 3a zeigt einen Querschnitt durch das Klemmgehäuse 2 im Schnitt B - B in Fig. 2, also nahe des oberen Endes. Fig. 3b zeigt den Ausschnitt D aus Fig. 3a. Der Ausschnitt D zeigt, dass die Kulisse 18 an dieser Stelle groß ist. Vorzugsweise hat die Kulisse 18 eine Breite von 1,38 mm und eine Höhe von 0,84 mm. Fig. 3c zeigt einen Querschnitt im Schnitt F - F von Fig. 2. Der Ausschnitt G in Fig. 3d zeigt, dass die Kulisse 18 auf dieser Höhe des Klemmgehäuses 2 kleiner ist als in Ausschnitt D. Vorzugsweise ist die Kulissenbreite hier 0,97 mm und die Höhe der Kulisse beträgt 0,66 mm. Fig. 3e zeigt einen Querschnitt durch das Klemmgehäuse 2 im Schnitt C - C. Fig. 3f zeigt einen Ausschnitt E aus der Fig. 3e und zeigt, dass die Kulisse 18 auf dieser Höhe des Klemmgehäuses 2 wiederum kleiner ist. Die Kulissenbreite beträgt vorzugsweise lediglich 0,53 mm und die Höhe der Kulisse 18 beträgt 0,31 mm. Die Figuren 3a - 3f zeigen, dass der Querschnitt der Kulisse zum unteren Ende hin abnimmt.

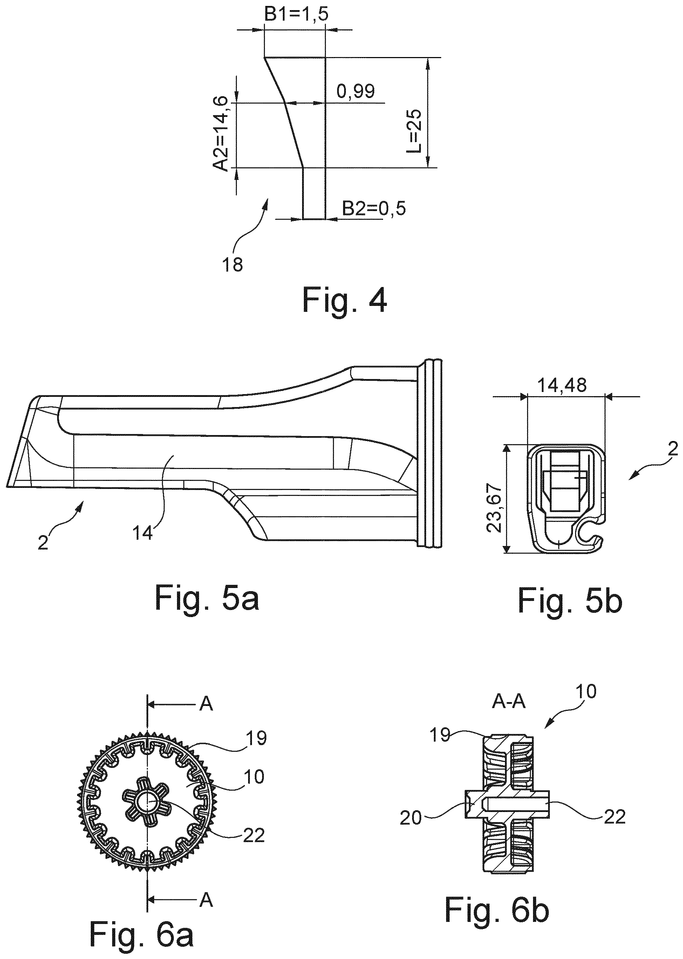

Fig. 4 zeigt die Kulissengeometrie mit Bemaßungen. Die Kulisse 18 hat im Wesentlichen die Form eines rechtwinkligen Trapezes. Dabei ist L die Länge der Kulisse 18, B1 ist die maximale Kulissenbreite und B2 ist die minimale Kulissenbreite. B1 ist dabei die Einstellung für die maximale Tropfrate und B2 ist die Einstellung für die minimale Tropfrate. Vorzugsweise beträgt B1 1,5 mm, B2 0,5 mm und L 25 mm. Die minimale Tropfrate ist null, d.h. keine Flüssigkeit fließt mehr durch den Schlauch 14. Die Flanke des Trapezes, die dem rechten Winkel gegenüberliegt, hat aber keine konstante Neigung. Die Flanke ist in zwei Abschnitte aufgeteilt, einen Abschnitt mit geringerer Tropfrate A2 und einen Abschnitt mit höherer Tropfrate A1. Der Abschnitt mit der höheren Tropfrate A1 ist der vordere Abschnitt, der näher an der maximalen Kulissenbreite B1 liegt und eine höhere Kulissenbreite hat. Der Abschnitt der geringeren Tropfrate A2 ist der hintere Abschnitt, der näher an der minimalen Kulissenbreite B2 liegt und die niedrigere Kulissenbreite aufweist. Zwischen den beiden Abschnitten A1, A2 liegt der Knickpunkt K. Der Knickpunkt K ist als der Punkt definiert, der zwischen den Abschnitten A1, A2 liegt. Die Kulissenbreite im Knickpunkt ist das Mittel von B1 und B2. Vorzugsweise ist der Abschnitt der niedrigen Tropfrate A2 14,6 mm lang und die Kulissenbreite im Knickpunkt beträgt 0,99 mm.

Aus Fig. 4 geht hervor, dass der Abschnitt der geringeren Tropfrate A2 länger als der Abschnitt der höheren Tropfrate A1 ist. A2 ist größer als A1. Da der Einstellweg der Tropfrate von der Länge der Kulisse abhängig ist, ist der Einstellweg der geringeren Tropfrate somit größer als der Einstellweg der höheren Tropfrate. Das ermöglicht dem Nutzer ein genaueres Einstellen einer geringeren Tropfrate. Durch die weniger steile Flanke bei dem Abschnitt der geringeren Tropfrate ist die Verstellkraft für die geringere Tropfrate geringer als die Verstellkraft für die höhere Tropfrate.

Der Bereich in dem die Feineinstellung stattfindet, ist entsprechend verlängert. Der Bereich mit niedrigerer Querschnittsbreite ist vorzugsweise 14,6 mm lang. Die damit einhergehende Querschnittsveränderung im Bereich vor und nach dem Knickpunkt führt zu einem angepassten Einstellverhalten. Eine weitere Verschiebung des Knickpunkts führt wiederum zu einer Verkürzung des Einstellbereichs bis 60 Tropfen/min. Der Durchfluss wird durch den ausgebildeten Fluidkanal im Schlauch bestimmt. Die Länge des Fluidkanals wird durch die Pressung des Rades auf den Schlauch bestimmt.

Im Fall des verwendeten Raddurchmessers (ca. 14,2 mm) und der vorgegebenen Pressung erfolgt der Radkontakt (Rad-Schlauch) über ca. 5 mm (Hertzsche Pressung). Versuche haben gezeigt, dass das Rädchen 10 nicht zu klein werden darf (>14,00 mm), da sonst das initiierte Fließen des Schlauchs 14 zu punktuell erzeugt wird (kein dichtes Schließen des Fluidkanals). Eine weitere Vergrößerung des Rädchens 10 erhöht die aufzunehmenden Kräfte im System und sollte daher 15,00 mm nicht überschreiten. Ansonsten müssen weiter Anpassungen der Wandstärke erfolgen, um die erhöhten Kräfte aufzunehmen. Ebenso führt eine Vergrößerung des Raddurchmessers zu einer Vergrößerung der Rollenklemme 1. Der mit dieser Situation hervorgerufen Überstand von 4 mm (+/- 1 mm) des Rädchens 10 über das Gehäuse ermöglicht eine gute Bedienbarkeit im Bereich der größten Kräfte. Für das Erreichen des Einstellverhaltens muss des Weiteren eine seitliche Fließbehinderung des Schlauches stattfinden (Bereich mit erhöhter Pressung gegenüber dem Hauptpressbereich).

Die Breite einer Nut der Lauffläche 8 darf nicht kleiner als 0,3 mm werden. Die Nut soll im Querschnitt annähernd rechtwinklig ausgeführt werden (90° +/-10°). Ein trapezförmiger Querschnitt ist auch zulässig.

Fig. 5a zeigt eine Seitenansicht des Klemmgehäuses 2. Dabei ist der Verlauf des Schlauches 14 innerhalb des Klemmgehäuses 2 angedeutet. Der Schlauch 14 wird am vorderen/ oberen Ende in das Klemmgehäuse 2 eingeführt und verlässt das Klemmgehäuse 2 am unteren Ende. Das Klemmgehäuse 2 ist vorzugsweise 53,65 mm lang. Fig. 5b zeigt einen Querschnitt durch das Klemmgehäuse 2 mit Bemaßungen. Das Klemmgehäuse 2 ist vorzugweise 23,67 mm hoch und 14,48 mm breit.

Fig. 6a zeigt das Rädchen 10 der Rollenklemme 1 in einer Draufsicht. Das Rädchen 10 ist vorzugweise aus Kunststoff beispielsweise durch Spritzguss gefertigt. Das Rädchen 10 weist auf der Umfangsfläche eine Rändelung 19 auf und ist somit griffig gestaltet. Fig. 6b zeigt eine Schnittansicht des Rädchens 10 durch den Schnitt A - A in Fig. 6a. Das Rädchen 10 hat beidseitig ausgebildete Achszapfen 21 im Zentrum, die in die Lauffläche 8 in den Seitenwänden 4 eingreifen. In einer Seite des Achszapfens 21 ist eine Bohrung 22 angebracht, die sich von einer Seite durch das Zentrum des Rädchens 10 erstreckt. Die Bohrung 22 verläuft dabei entlang einer Rotationsachse des Rädchens 10.

Das Rädchen 10 muss mit Stegen/Rippen/ der Rändelung 19 auf der Kontaktfläche zum Schlauch 14 versehen sein, da ansonsten das Fließen des Schlauches nicht ausreichend initiiert wird. Ein dichtes Schließen ist ansonsten nicht möglich. Ein glattes Rädchen 10 ist ungeeignet.

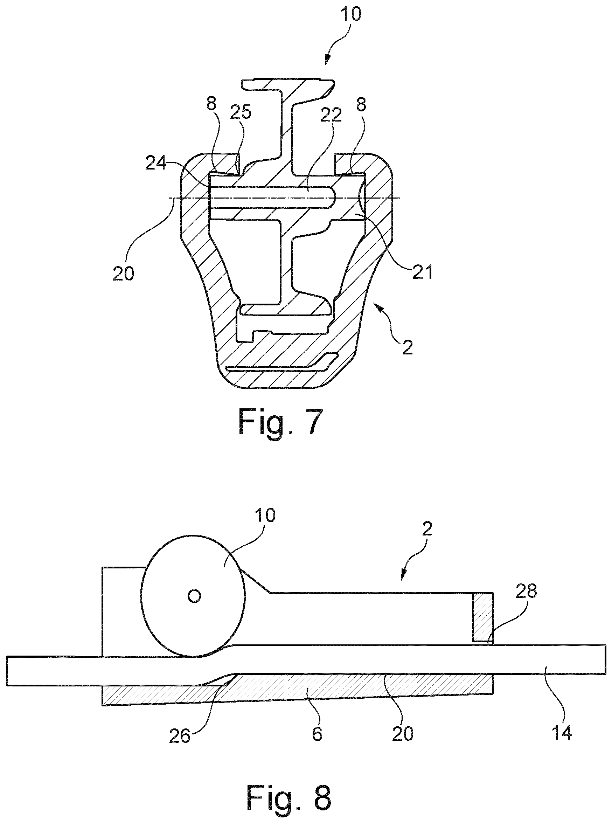

Fig. 7 zeigt einen Querschnitt durch das Klemmgehäuse 2 an der Stelle an der das Rädchen 10 an der Lauffläche 8 des Klemmgehäuses 2 aufliegt. Dabei ist zu sehen, dass zwischen dem Klemmgehäuse 2 bzw. der Lauffläche 8 und der Lauffläche 24 des Rädchens 10 eine Freistellung vorhanden ist. D.h. die Lauffläche 8 und das Rädchen berühren sich nicht über die gesamte Länge der Lauffläche 8, sondern nur in einer im Wesentlichen punktförmigen Abstütz-, Auflage- oder Kontaktstelle 25. Um die Freistellung zu realisieren, ist die Lauffläche 8 beispielsweise angestellt. Die Abstütz- oder Kontaktstelle 25 bzw. der Kontaktbereich zwischen der Lauffläche 8 des Klemmgehäuses 2 und dem Rädchen 10 ist durch die Neigung der Lauffläche 8 in Richtung einer Radmitte bzw. dem Gehäuseinneren verschoben. Dadurch ergibt sich die im Wesentlichen punktförmige Abstütz- oder Kontaktstelle 25 zwischen der Lauffläche 8 des Klemmgehäuses 2 und der Lauffläche 24 des Rädchens 10. Indem die Abstütz- oder Kontaktstelle 25 in Richtung der Radmitte verschoben wird, verringern sich die Kräfte beim Bewegen des Rädchens 10, die auf das Rädchen 10 bzw. die Achszapfen 21 des Rädchens 10 wirken.

Da das Rädchen 10 durch die Spritzgussoptimierung in den mechanischen Eigenschaften zum Teil etwas verschlechtert wurde, ist eine Anpassung in dem Klemmgehäuse 2 vorgenommen worden. Die Anpassung betrifft die Gestaltung der Kontaktfläche zwischen der Lauffläche 24 des Rädchens 10 und dem Klemmgehäuse 2. Der Kontaktbereich ist durch eine Freistellung der Lauffläche 24 maximal zum Radmittelpunkt verschoben. Dadurch wird die mögliche elastische Deformation in der Radachse reduziert und so der geringeren Steifigkeit der Radachse durch die Aushöhlung/ Bohrung 22 entgegengewirkt.

Die Tropfratenkonstanz ist ein komplexes Zusammenspiel aus den Deformationen des Klemmgehäuses 2, Rädchens 10 und des Schlauches 14. Die Elastizität im System muss gegenüber dem Schlauch möglichst geringgehalten werden, um eine hohe Tropfratenkonstanz zu erzeugen. Die erzielte Verbesserung der Steifigkeit des Gesamtsystems Rollenklemme 1, dient auch dem Ziel einer möglichst hohen Tropfratenkonstanz.

Fig. 8 zeigt eine schematische Darstellung von einer Arretierung der Rollenklemme 1 auf dem Schlauch 14. Um die Rollenklemme 1 auf dem Schlauch 14 festzustellen, beispielsweise im Auslieferzustand des Systems, wird die Rollenklemme 1 auf den Schlauch 14 geklemmt. Dazu wird der Schlauch 14 in der Rollenklemme 1 umgelenkt. Die erste Umlenkung ist durch eine Stufe 26 in dem Steg 6 realisiert. Durch die Stufe 26 würde der Schlauch noch oben gedrückt werden und die Kontur der Schlauchablagefläche 20 verlassen. Durch die Durchgangsöffnung 28 des Klemmgehäuses 2 ist das jedoch nicht möglich. Der Durchmesser der Durchgangsöffnung 28 ist nur unwesentlich größer als der Schlauchdurchmesser. Dadurch kann sich der Schlauch 14 zwar frei in seine Längsrichtung vor und zurückbewegen, kann sich aber nicht quer zu seiner Längsrichtung bewegen. Damit bilden die Stufe 26 und die Durchgangsöffnung 28 zusammen eine Umlenkung, die den Schlauch 14 in dem Klemmgehäuse 2 feststellt/ arretiert.

Die Arretierung der Rollenklemme 1 auf dem Schlauch 14 wurde bisher durch ein Deformieren des Schlauchquerschnitts durch das Rädchen 10 bewerkstelligt. Dies hatte zur Folge, dass auf dem Schlauch 14 eine Deformation durch die Rändelung 19 des Rädchens 10 stattfand. Das Konzept wurde dahingehend geändert, dass der Schlauch 14 im Wesentlichen umgelenkt und nicht mehr im Querschnitt deformiert wird. Für die Klemmung durch Umlenkung ist jedoch ein entsprechendes Wiederlager nötig. Daher wurde die Durchgangsöffnung 28 am unteren Ende der Rollenklemme 1 (rechts) auf ein Minimum reduziert. Durch die Anpassung des Konzeptes wurde der Effekt der Schlauchdeformation durch die Rändelung 19 stark reduziert und gleichzeitig die benötigte Verschiebekraft für die Positionierung der Rollenklemme 1 auf dem Schlauch 14 reduziert.

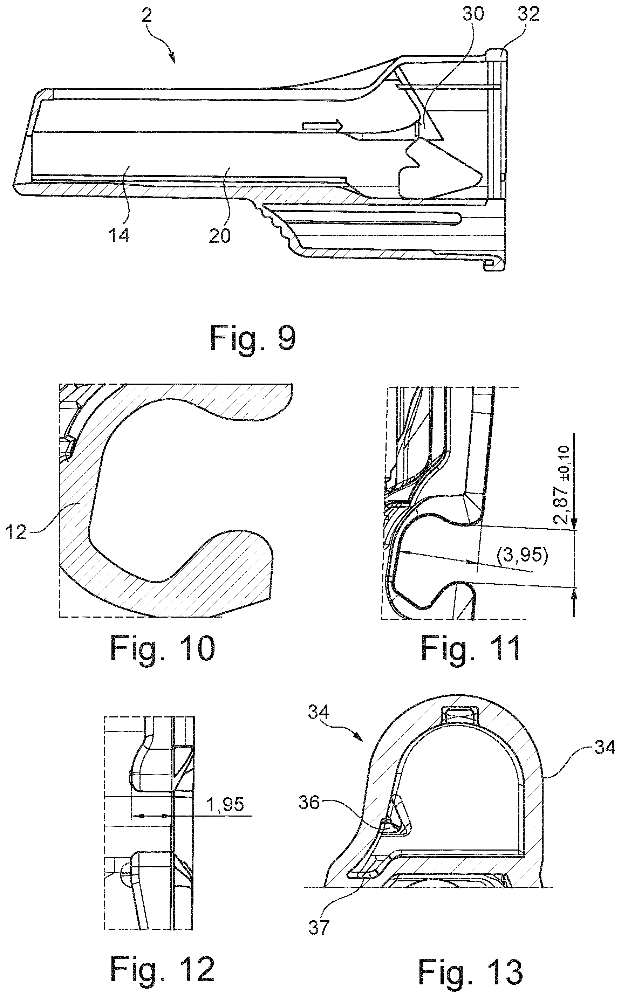

Fig. 9 zeigt eine Radhebekulisse 30 in der Seitenwand 4. Die Radhebekulisse 30 ermöglich es dem Rädchen 10, sich von der Schlauchauflagefläche 20 abzuheben. Ohne die Radhebekulisse 30 würde das Rädchen 10 beim Öffnen der Rollenklemme 1 der Schlauchauflagefläche 20 folgen. Durch die Pressung des Rädchens 10 auf den Schlauch 14 besteht die Möglichkeit, dass das Rädchen 10 unter einem Gehäusesteg 32 des Klemmgehäuses 2 aus dem Klemmgehäuse 2 gedrückt wird. Die Radhebekulisse 30 minimiert die Pressung des Rädchens 10 auf den Schlauch 14, indem es dem Rädchen 10 erlaubt die Kontur der Schlauchauflagefläche 20 nach oben in Pfeilrichtung in Fig. 9 zu verlassen.

Die Radabhebekulisse 30 ist in die Seitenwand 4 eingebracht. Die Radabhebekulisse 30 führt dazu, dass das Rollenklemmenrädchen 10 beim Öffnen der Rollenklemme 1 nicht der Kontur der Schlauchauflagefläche 20 folgen kann. In Konsequenz wird der Kontakt zum Schlauch 14 und damit die Möglichkeit des Rädchens 10, sich auf dem Schlauch 14 abzustützen reduziert.

Fig. 10 zeigt eine Seitenansicht des Schlauchhalters 12. Der Schlauchhalter 12 ist asymmetrisch. Der Schlauchhalter 12 ist ein Vorsprung aus einer Seite des Klemmgehäuses 2 und ist im Wesentlichen halbkreisförmig ausgebildet. Der Schlauchhalter 12 hat eine tiefere Einbuchtung an der Unterseite, in die der Schlauch fest eingedrückt wird. Die Oberseite des Schlauchhalters 12 ist nicht so tief eingebuchtet wie die Unterseite. Dadurch ist der Schlauchhalter 12 asymmetrisch. Die Geometrie wurde dahingehend optimiert, einen festen Sitz bei einfachem Eindrücken und Herausziehen des Schlauches 14 zu ermöglichen. Um dieses Verhalten zu erzielen muss die Öffnung in einem Bereich von 2,9+/-0,15 mm liegen. Die Breite bzw. der Umfang müssen dem Durchmesser des Schlauches 14 angepasst werden. Fig. 11 zeigt die Schlauchhaltergeometrie. Bei einem Schlauch 14 von 4,1 mm Nenndurchmesser beträgt der Umfang 13,9 +/-1,5 mm. Die Länge der Halterung muss min. 1,8 mm betragen (1,95 +1,0/-0,15). Die Geometrie auf der Oberseite (rechts) wurde hinsichtlich dem Werkzeugkonzept und einer möglichen Beschädigung der Verpackung optimiert. In Fig. 12 ist gezeigt, dass die Länge des Schlauchhalters 12 1,95 mm beträgt.

Fig. 13 zeigt einen Querschnitt durch den sogenannten Dornschutz 34, wie er vorstehend bereits erwähnt wurde. Der Dornschutz 34 besteht im Wesentlichen aus einem Halbbogen/U-Profil 35, der mit seinen freien Längskanten an der Unterseite des Stegs 6 stoffeinstückig angeschlossen ist, um ein geschlossenes Querschnittsprofil zu bilden. Der Halbbogen/U-Profil weist nahe an der breitesten Stelle/entfernt zum Scheitelpunkt des U-Profils nahe einer der freien Längskanten eine innere elastische Rippe 36 auf. Die Rippe 36 ist an einer inneren Ausbuchtung 37 des Dornschutzes 34 ausgebildet, die sich in Richtung des Klemmgehäuses 2 erstreckt. Die Ausbuchtung ist dabei in den Steg 6 eingearbeitet. Ein Dorn am Ende des Schlauches 14 kann in den Dornschutz 34 in Längsrichtung des Gehäuses 2 gesteckt werden und wird durch die Rippe 36 derart eingeklemmt, dass der Dorn im Dornschutz 34 verbleibt. Der Dornschutz 34 wird beispielsweise genutzt, wenn der Schlauch 14 vom Patienten entfernt wird. Bei der Entsorgung des Infusionsbestecks wird der Dorn des Schlauches in den Dornschutz 34 eingesteckt/geklemmt, damit sich der Nutzer nicht an dem Dorn verletzt.

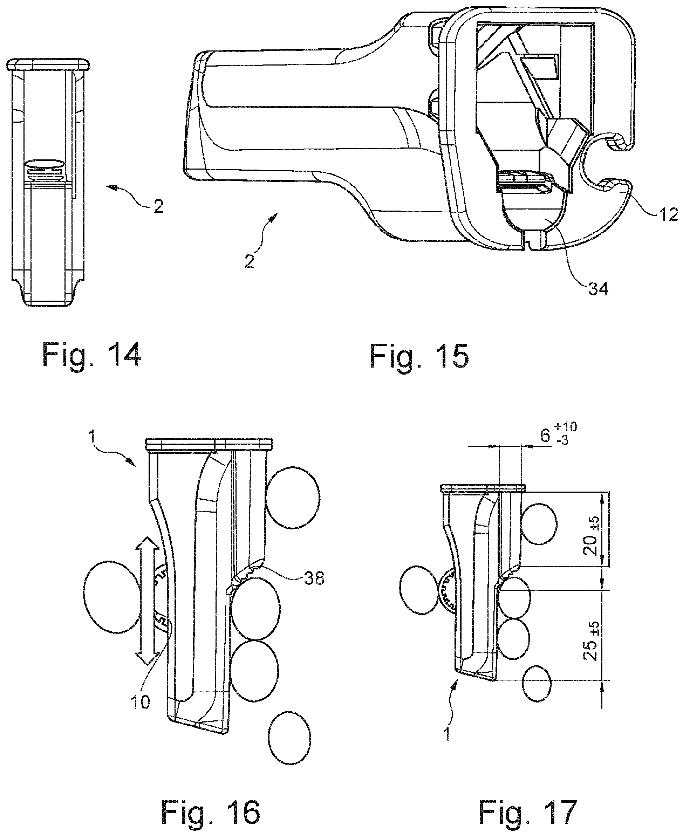

Im Stand der Technik wurde der Dornschutz durch eine Schlitzung in der Gehäuseunterseite realisiert. Die Fertigung des Schlitzes verschleißt das Werkzeug, mit dem das Klemmgehäuse gefertigt wird. Weiterhin muss eine Dichtfläche für die Schlitzung vorhanden sein. Der offenbarungsgemäße Dornschutz 34 hingegen ist fester als der herkömmliche Dornschutz. Fig. 14 zeigt die Unterseite des Klemmgehäuses 2. Dabei ist zu sehen, dass die Schlitzung im Klemmgehäuse 2 entfällt.

Fig. 15 zeigt eine isometrische Darstellung des oberen Endes des Klemmgehäuses 2. Darauf ist auch der Dornschutz 34 abgebildet.

Fig. 16 zeigt eine schematische Darstellung von einer Hand (fünf symbolisch dargestellte Fingerspitzen), die die Rollenklemme 1 umgreift. Demnach greift ein Nutzer die Rollenklemme 1 derart, dass der Nutzer mit dem Daumen das Rollenklemmenrädchen 10 verstellen kann. Das Klemmgehäuse 2 weist eine Stufe 38 etwa auf halber Höhe/Länge des Klemmgehäuses 2 auf. Diese Stufe 38 ist ausgebildet, dass der Nutzer die Rollenklemme 1 ergonomisch in der Hand halten kann. Die Stufe 38 auf ca. der Mitte der Rollenklemmenrückseite erzeugt ein ergonomisches Halten der Rollenklemme 1 und ein einfaches Schließen. Bezogen auf die Größe wird derart ein optimales Zusammenspiel erzeugt.

Fig. 17 zeigt eine Seitenansicht der Rollenklemme 1 mit Bemaßungen. Dabei ist zu erkennen, dass der obere Teil der Rollenklemme 1 bis zur Stufe 20 mm lang ist. Der untere Teil von der Stufe bis zum unteren Ende ist 25 mm lang. Beide Maße haben jeweils eine Toleranz von +-0,5 mm. Die Stufe ist 6 mm hoch. Die Toleranz der Stufe ist vorzugsweise nach oben 0,6 mm und nach unten 0,3 mm.

## Patentansprüche

1. Infusionsbesteck mit einem Schlauch (14) und einer zur Regulierung eines Fluiddurchflusses durch den Schlauch (14) vorgesehenen und ausgebildeten Rollenklemme (1), wobei die Rollenklemme (1) aufweist:
- ein im Querschnitt U-/ oder C-förmiges Gehäuse (2), das Seitenwände (4) und einen die Seitenwände (4) verbindenden Steg (6) aufweist, auf dem der Schlauch (14) aufliegt; und
- ein Rädchen (10), das an Laufflächen (8) in oder an den Seitenwänden (4) des Gehäuses (2) drehbar und in Längsrichtung des Gehäuses (2) verschiebbar abgestützt ist, wofür die Laufflächen (8) dem Steg (6) zugewandt sind und sich in ihrer Breite in Gehäusequerrichtung sowie längs in Gehäuselängsrichtung derart erstrecken, dass eine Bewegung des Rädchens (10) längs der Laufflächen (8) einen Querschnitt des Schlauchs (14) verändert,
**dadurch gekennzeichnet, dass**
die Laufflächen (8) zu einer Rotationsachse des Rädchens (10) angestellt sind, sodass die Laufflächen (8) jeweils eine im Wesentlichen punktförmige Abstütz- oder Kontaktstelle (25) mit einer Radachse des Rädchens (10) bilden, die an einem, in Breitenrichtung gesehen, einer Radmitte zugewandten, Innenabschnitt der Radachse ausgebildet ist.

2. Infusionsbesteck nach Anspruch 1, **dadurch gekennzeichnet, dass** die Radachse konisch ausgebildet ist, sodass die Abstütz- oder Kontaktstelle (25) in dem Innenabschnitt der jeweiligen Lauffläche (8) ausgebildet ist.

3. Infusionsbesteck nach Anspruch 1 oder 2, **dadurch gekennzeichnet, dass** die Radachse hohl ist, insbesondere eine Bohrung (22) entlang einer Rotationsachse des Rädchens (10) in einem beidseitig ausgebildeten Achszapfen (21) des Rädchens (10) vorhanden ist.

4. Infusionsbesteck nach einem der Ansprüche 1 bis 3, **dadurch gekennzeichnet, dass** die Abstütz- oder Kontaktstelle (25), an einem, in Breitenrichtung gesehen, maximal innersten Abschnitt der Laufflächen (8) ausgebildet ist.

5. Infusionsbesteck nach einem der Ansprüche 1 bis 4, **dadurch gekennzeichnet, dass** das Rädchen (10) bei einer Längsbewegung den Schlauch (14) in eine in Längsrichtung des Gehäuses (2) schmaler und/oder flacher werdende Kulisse (18) in dem Steg (6) presst, wodurch eine Pressung des Rädchens (10) auf den Schlauch (14) zunimmt und ein Querschnitt des Schlauchs (14) abnimmt.

6. Infusionsbesteck nach Anspruch 5, **dadurch gekennzeichnet, dass** die Kulisse (18) einen Längsabschnitt einer tendenziell geringeren Schlauchpressung (A1) und einen Längsabschnitt einer tendenziell größeren Schlauchpressung (A2) aufweist und der Abschnitt der tendenziell größeren Schlauchpressung (A2) länger ist als der Abschnitt der tendenziell geringeren Schlauchpressung (A1).

7. Infusionsbesteck nach Anspruch 6, **dadurch gekennzeichnet, dass** eine Kulissenbreite mit der kleinsten Schlauchpressung größer als eine Kulissenbreite der größten Schlauchpressung ist.

8. Infusionsbesteck nach einem der Ansprüche 1 bis 7, **dadurch gekennzeichnet, dass** der Steg (6) eine vorzugsweise durch eine im Steg (6) ausgebildete Stufe definierte, sowie in Querrichtung des Gehäuses (2) verlaufende Kante (26) aufweist, wodurch der Schlauch (14) beim Arretieren der Rollenklemme (1) auf dem Schlauch (14) mittels des Rädchens (10) sowohl an der Kante (26) als auch an einem dem Steg (6) gegenüberliegenden Randabschnitt einer Durchgangsöffnung (28) anliegt und somit eingeklemmt ist.

9. Infusionsbesteck nach Anspruch 8, **dadurch gekennzeichnet, dass** die Kante (26) mit Blickrichtung hin zur Durchgangsöffnung (28) ausgebildet ist.

10. Infusionsbesteck nach Anspruch 8 oder 9, **dadurch gekennzeichnet, dass** der Durchmesser der Durchgangsöffnung (28) des Klemmgehäuses (2) im Wesentlichen dem Durchmesser des Schlauchs (14) entspricht.

11. Infusionsbesteck nach einem der Ansprüche 8 bis 10, **dadurch gekennzeichnet, dass** das Rädchen (10) über einen vordefinierten Verschiebeweg in Längsrichtung verschiebbar ist und die Kante (26) in einem Mittenabschnitt des Verschiebewegs positioniert ist.

12. Infusionsbesteck nach einem der Ansprüche 1 bis 11, **dadurch gekennzeichnet, dass** das Klemmgehäuse (2) einen asymmetrischen Schlauchhalter (12) aufweist.

13. Infusionsbesteck nach einem der Ansprüche 1 bis 12, **dadurch gekennzeichnet, dass** das Klemmgehäuse (2) eine Rippe (36) im Inneren des Klemmgehäuses (2) aufweist, um einen Dorn einzuklemmen.

14. Infusionsbesteck nach einem der Ansprüche 1 bis 13, **dadurch gekennzeichnet, dass** in der Seitenwand (4) eine Radabhebekulisse (30) eingebracht ist, die es dem Rädchen (10) ermöglicht, sich von einer Schlauchauflagefläche (20) abzuheben.

## Claims

1. An infusion set having a tube (14) and a roller clamp (1) provided and designed to regulate the flow of fluid through the tube (14), the roller clamp (1) comprising:
- a housing (2) which is U-shaped or C-shaped in cross-section and has side walls (4) and a crosspiece (6) which connects the side walls (4) and on which the tube (14) rests; and
- a small wheel (10) which is supported on running surfaces (8) in or on the side walls (4) of the housing (2) so as to be rotatable and to be displaceably supported in the longitudinal direction of the housing (2), for which purpose the running surfaces (8) face the crosspiece (6) and the widths of the running surfaces extend in the transverse direction of the housing and lengthwise in the longitudinal direction of the housing so that a movement of the small wheel (10) along the running surfaces (8) alters a cross-section of the tube (14),
**characterized in that**
the running surfaces (8) are positioned at an angle to a rotational axis of the small wheel (10) such that the running surfaces (8) each form a substantially punctiform support or contact point (25) with a wheel axle the small wheel (10), which is formed on an inner portion of the wheel axle facing the wheel center, viewed in the width direction.

2. The infusion set according to claim 1, **characterized in that** the wheel axle is formed conically, so that the support or contact point (25) is formed in the inner portion of the respective running surface (8).

3. The infusion set according to claim 1 or 2, **characterized in that** the wheel axle is hollow, particularly a bore (22) along an axis of rotation of the small wheel (10) is provided in a trunnion (21) of the small wheel (10) formed on both sides.

4. The infusion set according to any of claims 1 to 3, **characterized in that** the support or contact point (25) is formed on a maximally innermost portion of the running surfaces (8), when viewed in the width direction.

5. The infusion set according to any of claims 1 to 4, **characterized in that**, during longitudinal movement, the small wheel (10) presses the tube (14) into a motion link (18) provided in the crosspiece (6) which becomes narrower and/or flatter in the longitudinal direction of the housing (2), thereby causing a pressing of the small wheel (10) onto the tube (14) to increase, and a cross-section of the tube (14) to decrease.

6. The infusion set according to claim 5, **characterized in that** the motion link (18) has a longitudinal section of a tendentially smaller tube pressing (A1) and a longitudinal section of a tendentially larger tube pressing (A2), and the section of the tendentially larger tube pressing (A2) is longer than the section of the tendentially smaller tube pressing (A1).

7. The infusion set according to claim 6, **characterized in that** a motion link width of the smallest tube pressing is larger than a motion link width of the largest tube pressing.

8. The infusion set according to any of claims 1 to 7, **characterized in that** the crosspiece (6) includes an edge (26) preferably defined by a step formed in the crosspiece (6) and extending in the transverse direction of the housing (2), whereby, when the roller clamp (1) is locked on the tube (14) by means of the small wheel (10), the tube (14) abuts both on the edge (26) and on an edge portion of a through-opening (28) facing the crosspiece (6) and is thus clamped.

9. The infusion set according to claim 8, **characterized in that** the edge (26) is formed with view towards the through-opening (28).

10. The infusion set according to claim 8 or 9, **characterized in that** the diameter of the through-opening (28) of the clamp housing (2) corresponds substantially to the diameter of the tube (14).

11. The infusion set according to any of claims 8 to 10, **characterized in that** the small wheel (10) is displaceable in the longitudinal direction over a predefined displacement path and the edge (26) is positioned in a central portion of the displacement path.

12. The infusion set according to any of claims 1 to 11, **characterized in that** the clamp housing (2) includes an asymmetrical tube holder (12).

13. The infusion set according to any of claims 1 to 12, **characterized in that** the clamp housing (2) includes a rib (36) inside the clamp housing (2) so as to clamp a spike.

14. The infusion set according to any of claims 1 to 13, **characterized in that** a wheel lifting motion link (30) which enables the small wheel (10) to lift off the tube bearing surface (20) is inserted in the side wall (4).

## Revendications

1. Ensemble de perfusion avec un tuyau (14) et une pince à rouleaux (1) configurée et prévue pour la régulation d'un débit fluidique à travers le tuyau (14), dans lequel la pince à rouleaux (1) présente :
- un boîtier (2) en forme de U et/ou de C en section qui présente des parois latérales (4) et une nervure (6) reliant les parois latérales (4), sur laquelle repose le tuyau (14) ; et
- une roulette (10) qui est en appui de manière rotative sur des surfaces de roulement (8) dans ou au niveau des parois latérales (4) du boîtier (2) et de manière déplaçable dans le sens longitudinal du boîtier (2) pour lequel les surfaces de roulement (8) sont tournées vers la nervure (6) et s'étendent dans leur largeur dans le sens transversal du boîtier ainsi que longitudinalement dans le sens longitudinal du boîtier de telle manière qu'un mouvement de la roulette (10) modifie une section du tuyau (14) le long des surfaces de roulement (8),
**caractérisé en ce que**
les surfaces de roulement (8) sont mises en place par rapport à un axe de rotation de la roulette (10) de sorte que les surfaces de roulement (8) forment respectivement un point d'appui ou de contact (25) sensiblement ponctuel avec un essieu de roue de la roulette (10) qui est configuré au niveau d'une section intérieure de l'essieu de roue, vu dans le sens de la largeur, tournée vers un milieu de roue.

2. Ensemble de perfusion selon la revendication 1, **caractérisé en ce que** l'essieu de roue est configuré de manière conique de sorte que le point d'appui ou de contact (25) soit configuré dans la section intérieure de la surface de roulement (8) respective.

3. Ensemble de perfusion selon la revendication 1 ou 2, **caractérisé en ce que** l'essieu de roue est creux, en particulier un alésage (22) est présent le long d'un axe de rotation de la roulette (10) dans un tourillon (21) de la roulette (10) configuré des deux côtés.

4. Ensemble de perfusion selon l'une quelconque des revendications 1 à 3, **caractérisé en ce que** le point d'appui ou de contact (25) est configuré au niveau d'une section la plus intérieure au maximum des surfaces de roulement (8), vu dans le sens de la largeur.

5. Ensemble de perfusion selon l'une quelconque des revendications 1 à 4, **caractérisé en ce que** la roulette (10) presse lors d'un mouvement longitudinal le tuyau (14) dans une coulisse (18) devenant plus étroite et/ou plate dans le sens longitudinal du boîtier (2) dans la nervure (6), selon lequel un pressage de la roulette (10) augmente sur le tuyau (14) et une section du tuyau (14) diminue.

6. Ensemble de perfusion selon la revendication 5, **caractérisé en ce que** la coulisse (18) présente une section longitudinale d'un pressage de tuyau (A1) généralement plus faible et une section longitudinale d'un pressage de tuyau (A2) généralement plus important et la section du pressage de tuyau (A2) généralement plus important est plus longue que la section du pressage de tuyau (A1) généralement plus faible.

7. Ensemble de perfusion selon la revendication 6, **caractérisé en ce qu'**une largeur de coulisse avec le pressage de tuyau le plus petit est plus grande qu'une largeur de coulisse avec le pressage de tuyau le plus important.

8. Ensemble de perfusion selon l'une quelconque des revendications 1 à 7, **caractérisé en ce que** la nervure (6) présente une arête (26) définie de préférence par un gradin configuré dans la nervure (6), ainsi que s'étendant dans le sens transversal du boîtier (2), selon lequel le tuyau (14) repose lors de l'arrêt de la pince à rouleaux (1) sur le tuyau (14) au moyen de la roulette (10) non seulement sur l'arête (26) mais aussi sur une section de bord d'une ouverture traversante (28) opposée à la nervure (6) et est ainsi serré.

9. Ensemble de perfusion selon la revendication 8, **caractérisé en ce que** l'arête (26) est configurée face à l'ouverture traversante (28).

10. Ensemble de perfusion selon la revendication 8 ou 9, **caractérisé en ce que** le diamètre de l'ouverture traversante (28) du boîtier de serrage (2) correspond sensiblement au diamètre du tuyau (14).

11. Ensemble de perfusion selon l'une quelconque des revendications 8 à 10, **caractérisé en ce que** la roulette (10) est déplaçable sur une voie de déplacement prédéfinie dans le sens longitudinal, et l'arête (26) est positionnée dans une section médiane de la voie de déplacement.

12. Ensemble de perfusion selon l'une quelconque des revendications 1 à 11, **caractérisé en ce que** le boîtier de serrage (2) présente un support de tuyau (12) asymétrique.

13. Ensemble de perfusion selon l'une quelconque des revendications 1 à 12, **caractérisé en ce que** le boîtier de serrage (2) présente une ailette (36) à l'intérieur du boîtier de serrage (2) afin de serrer une épine.

14. Ensemble de perfusion selon l'une quelconque des revendications 1 à 13, **caractérisé en ce qu'**une coulisse de levage de roue (30) est introduite dans la paroi latérale (4), coulisse qui permet à la roulette (10) de se lever d'une surface d'appui de tuyau (20).
